# EUROPÄISCHE PATENTSCHRIFT

(11) **EP 2 822 507 B1**
(45) Veröffentlichungstag und Bekanntmachung des Hinweises auf die Patenterteilung: **10.05.2017**
(21) Anmeldenummer: 13707385.4
(22) Anmeldetag: 04.03.2013
(51) Int. Cl.: A61F 2/30, A61F 2/36, A61F 2/38

(54) **PROTHESENTEIL**
PROSTHETIC PART
PARTIE DE PROTHÈSE

(30) Priorität: 09.03.2012 EP 12158698
(43) Veröffentlichungstag der Anmeldung: 14.01.2015
(73) Patentinhaber: Waldemar Link GmbH & Co. KG, 22339 Hamburg (DE)
(72) Erfinder: IREDI, Marco, 22848 Norderstedt (DE); STANGEL, Melanie, 22844 Norderstedt (DE)
(74) Vertreter: Hoffmann Eitle
(86) Internationale Anmeldenummer: PCT/EP2013/054252
(87) Internationale Veröffentlichungsnummer: WO 2013/131845

(56) Entgegenhaltungen:
- EP-A1- 0 474 015
- WO-A1-2010/020429
- DE-A1- 3 336 004
- US-A1- 2003 055 508

## Beschreibung

Die Erfindung betrifft ein Prothesenteil und insbesondere dessen Ausgestaltung zur Verbindung mit einem weiteren Prothesenteil.

Es ist bekannt, dass Knochen und Gelenke eines Menschen bei Beschädigungen, bspw. aufgrund von Krankheit oder Unfällen, durch Prothesen ersetzt werden können. Dadurch ist es möglich, dass ein Patient die ursprüngliche Funktionalität seines Knochenbaus zurückerhält. Da entsprechende Prothesen in der Regel dauerhaft im Körper des Patienten verbleiben, spricht man auch von Endoprothesen.

Entsprechende Prothesen bestehen häufig aus zwei oder mehr Einzelteilen, die je nach Bedarf zusammengesetzt werden können. So kann eine Prothese bspw. aus einem Prothesenhauptteil und einem Prothesenkopf bestehen. Solche mehrteiligen Prothesen bieten den Vorteil, dass verhältnismäßig wenige, untereinander austauschbare Einzelteile umfangreich miteinander kombiniert werden können, um so eine für einen bestimmten Patienten geeignete Prothese zu schaffen. Eine Prothese muss demnach nicht speziell für einen Patienten angefertigt werden, sondern kann auch in situ, d. h. sogar während des chirurgischen Eingriffs zur Implantation einer Prothese, auf die patientengemäßen Verhältnisse angepasst werden.

Außerdem wird durch die Möglichkeit des Zusammensetzens einer Prothese aus mehreren Prothesenteilen in situ erreicht, dass die chirurgischen Vorbereitungen zur Implantation einer Prothese weniger radikal erfolgen können. Im Zweifelsfall muss nämlich lediglich das Knochenmaterial entfernt werden, an dessen Stelle die einzelnen Prothesenteile treten. Ein zusätzlicher Knochenabtrag, um Teile einer Prothese durch Knochenbereiche durchführen zu können, in denen letztendlich keine oder andere, ggf. kleinere Teile der Prothese im implantierten Zustand zu liegen kommen, kann häufig vermieden werden. Die einzelnen Prothesenteile können nämlich in der Regel getrennt voneinander implantiert und erst anschließend miteinander verbunden werden. Indem der ausführende Chirurg anstelle einer einzigen, ggf. schwer handhabbaren Prothese eine Vielzahl von kleineren, in der Regel gut handhabbaren Prothesenteilen implantieren und erst anschließend verbinden muss, wird seine Arbeit deutlich erleichtert.

Im Stand der Technik sind verschiedene Möglichkeiten bekannt, einzelne Prothesenteile sicher zu einer einzigen Prothese miteinander zu verbinden. So ist bspw. bekannt, dass ein Prothesenteil einen konusförmigen Vorsprung zur Verbindung mit einem weiteren Prothesenteil mit einer komplementären konusförmigen Bohrung aufweist. Es ist weiterhin bekannt, dass entsprechende Konusverbindungen durch Sicherungsschrauben vor einem ungewollten Lösen gesichert werden. Dabei wird die Sicherungsschraube durch eine Bohrung des einen Prothesenteils geführt und greift wenigstens teilweise in eine Vertiefung oder Bohrung am anderen Prothesenteil ein.

Die Merkmale des Oberbegriffs des Anspruchs 1 sind aus EP-A-0474015 oder WO-A-2010/020429 bekannt.

Aufgabe der vorliegenden Erfindung ist es, Konusverbindungen von Prothesenteilen und Prothesen zu verbessern.

Diese Aufgabe wird durch ein Prothesenteil, eine Prothese sowie einen Satz umfassend wenigstens drei Prothesenteile gemäß den Ansprüchen 1 und 7 gelöst. Vorteilhafte Weiterbildungen ergeben sich aus den weiteren abhängigen Ansprüchen.

Demnach betrifft die Erfindung ein Prothesenteil mit einem konusförmigen Vorsprung zur Verbindung mit einem weiteren Prothesenteil mit einer komplementären konusförmigen Bohrung, wobei der konusförmige Vorsprung eine seitliche Ausnehmung für den Eingriff eines Sicherungselementes aufweist und das Prothesenteil mit dem konusförmigen Vorsprung einen abnehmbaren Konusaufsatz aufweist, der eine seitliche Ausnehmung für den Eingriff eines Sicherungselementes aufweist.

Weiterhin beschrieben wird eine Prothese umfassend einen erfindungsgemäßen Prothesenteil mit einem konusförmigen Vorsprung, einen Prothesenteil mit einer komplementären konusförmigen Bohrung und ein Sicherungselement, wobei der Prothesenteil mit der konusförmigen Bohrung eine Durchgangsöffnung zur Durchführung des Sicherungselements aufweist, sodass das Sicherungselement mit der seitlichen Ausnehmung des abnehmbaren Konusaufsatzes des, in das Prothesenteil mit der konusförmigen Bohrung eingesetzten Prothesenteils mit dem konusförmigen Vorsprung in Eingriff bringbar ist, wobei das Sicherungselement eine Madenschraube ist, deren Länge so gewählt ist, dass sie bei Fehlen des abnehmbaren Konusaufsatzes oder des Prothesenteils mit konusförmigem Vorsprung aus der Durchgangsöffnung zur Durchführung des Sicherungselementes ins Innere des Prothesenteils mit konusförmiger Bohrung fallen kann.

Außerdem betrifft die Erfindung einen Satz umfassend ein erstes erfindungsgemäßes Prothesenteil mit konusförmigen Vorsprung, ein zweites und ein drittes Prothesenteil mit jeweils einer komplementären konusförmigen Bohrung, wobei des zweite Prothesenteil eine Durchgangsöffnung zur Durchführung eines Sicherungselementes aufweist, sodass das Sicherungselement mit der seitlichen Ausnehmung des abnehmbaren Konusaufsatzes des, in das zweite Prothesenteil eingesetzten ersten Prothesenteils in Eingriff bringbar ist, und wobei das dritte Prothesenteil eine Durchgangsöffnung zur Durchführung eines Sicherungselementes aufweist, sodass das Sicherungselement mit der seitlichen Ausnehmung des konusförmigen Vorsprungs des, in das dritte Prothesenteil eingesetzten ersten Prothesenteils in Eingriff bringbar ist.

Indem der erfindungsgemäße Prothesenteil als Eingriffsmöglichkeit für ein Sicherungselement neben einer seitlichen Ausnehmung am konusförmigen Vorsprung auch noch eine seitliche Ausnehmung an einem abnehmbaren Konusaufsatz aufweist, ist er äußerst flexibel mit weiteren Prothesenteilen kombinierbar.

So lässt sich der erfindungsgemäße Prothesenteil bspw. mit einem Prothesenteil kombinieren, bei dem die konusförmige Bohrung dünnwandig ausgestaltet ist, d. h. die Bohrung umgebende Wand ist dünn. Bei einer konusförmigen und dünnwandig ausgestalteten Bohrung kann in der Regel keine Durchgangsöffnung vorgesehen werden, durch die ein Sicherungselement zum Eingriff mit einer seitlichen Ausnehmung an einem Prothesenteil mit konusförmigen Vorsprung geführt werden kann. Bei einer entsprechenden Durchgangsöffnung wäre nämlich nicht ausreichend sichergestellt, dass sie auch bei Beanspruchung der Konusverbindung auf Zug nicht ausreißt. Für den Fall einer dünnwandig ausgestalteten, konusförmigen Bohrung kann bei dem erfindungsgemäßen Prothesenteil das Sicherungselement auch in die seitliche Ausnehmung am Konusaufsatz des erfindungsgemäßen Prothesenteils eingreifen. Das Sicherungselement muss dann nicht mehr durch dünnwandige Bereiche des weiteren Prothesenteils geführt werden. Vielmehr kann die dafür vorgesehene Durchgangsöffnung durch Bereiche abseits der konusförmige Bohrung geführt werden, die in der Regel nicht dünnwandig sind. Die Sicherung der Konusverbindung kann so auch bei Prothesenteilen mit dünnwandig ausgestalteten, konusförmigen Bohrungen gewährleistet werden.

Für den Fall, dass der erfindungsgemäße Prothesenteil mit einem weiteren Prothesenteil verbunden werden soll, bei dem die konusförmige Bohrung dickwandig ausgeführt ist, kann ein, durch eine Durchgangsöffnung an diesem weiteren Prothesenteil geführtes Sicherungselement direkt in die seitliche Ausnehmung am konusförmigen Vorsprung eingreifen. "Dickwandig" in diesem Zusammenhang bedeutet, dass die Wand an der Stelle einer Durchgangsöffnung zur Durchführung eines Sicherungselementes eine ausreichende Wandstärke besitzt, sodass unter der zu erwartenden Maximalbelastungen (ggf. zzgl. einem Sicherheitsfaktor) ein Ausreißen der Durchgangsöffnung nicht zu erwarten ist.

Bevor das erfindungsgemäße Prothesenteil und ein weiteres Prothesenteil mit dickwandig ausgeführten, konusförmigen Bohrung miteinander verbunden werden, kann vorher der Konnusaufsatz vom erfindungsgemäßen Prothesenteil entfernt werden. Da der Konusaufsatz abnehmbar ausgebildet ist, ist dies ohne Weiteres möglich. Das Abnehmen des Konusaufsatzes ermöglicht, das erfindungsgemäße Prothesenteil auch mit solchen weiteren Prothesenteilen zu verwenden, bei denen kein Hohlraum zur Aufnahme des Konusaufsatzes vorgesehen ist oder ein entsprechender Hohlraum aufgrund der Ausgestaltung des weiteren Prothesenteil auch nicht geschaffen werden kann.

Das erfindungsgemäße Prothesenteil lässt sich also mit einer Vielzahl von weiteren Prothesenteiltypen verbinden. Das erfindungsgemäße Prothesenteil ermöglicht dabei, dass unabhängig von der Ausgestaltung des weiteren Prothesenteils eine Sicherung durch ein Eingreifen eines Sicherungselementes in eine seitliche Ausnehmung gewährleistet ist. Indem das erfindungsgemäße Prothesenteil verschiedentliche Sicherungen einer Konusverbindung ermöglicht, kann es äußerst flexibel eingesetzt und mit weiteren Prothesenteilen kombiniert werden.

Die seitliche Ausnehmung für den Eingriff eines Sicherungselementes am konusförmigen Vorsprung des erfindungsgemäßen Prothesenteils kann vorzugsweise senkrecht zur Konusachse ausgerichtet sein. Die Ausnehmung kann bspw. als umlaufende Vertiefung bzw. Nut, aber auch als Sackloch ausgebildet sein.

Der Konusaufsatz ist vorzugsweise rotationssymmetrisch, weiter vorzugsweise zylindrisch geformt. Die Ausnehmung am Konusaufsatz ist vorzugsweise senkrecht zu dessen Achse ausgerichtet und kann bspw. als umlaufende Vertiefung bzw. Nut oder als Sackloch ausgebildet sein. Es ist bevorzugt, wenn die Symmetrieachse des Konusaufsatzes im zusammengesetzten Zustand mit der Achse des konusförmigen Vorsprungs zusammenfällt.

Vorzugsweise ist der Konusaufsatz über eine Schraubenverbindung abnehmbar mit dem konusförmigen Vorsprung verbunden. Eine Schraubenverbindung bietet den Vorteil einer sicheren Verbindung bei gleichzeitiger einfacher Lösbar- bzw. Abnehmbarkeit des Konusaufsatzes vom konusförmigen Vorsprung. Der Konusaufsatz kann einen schraubenförmigen Fortsatz, der konusförmige Vorsprung ein Sackloch mit Innengewinde zur Bildung der Schraubenverbindung aufweisen.

Die Erfindung betrifft weiterhin eine Prothese, die neben dem zuvor beschriebenen Prothesenteil noch einen weiteren Prothesenteil umfasst. Dieser weitere Prothesenteil weist eine konusförmige Bohrung auf, die zum konusförmigen Vorsprung des erstgenannten Prothesenteils komplementär ist. In anderen Worten sind die beiden Prothesenteile zur Verbindung durch eine Konusverbindung ausgebildet.

In dem weiteren Prothesenteil ist eine Durchgangsöffnung zur Durchführung eines Sicherungselementes vorgesehen, die derart angeordnet ist, dass ein dort hindurchgeführtes Sicherungselement grundsätzlich in die seitliche Ausnehmung am Konusaufsatz des anderen Prothesenteils eingreifen kann, sofern die beiden Prothesenteile miteinander verbunden sind.

Bei dem Sicherungselement handelt es sich bevorzugt um eine Madenschraube. Zur Führung der Madenschraube kann die Durchgangsöffnung im Prothesenteil mit der konusförmigen Bohrung wenigstens teilweise ein Innengewinde aufweisen, in das das Außengewinde der Madenschraube eingreifen kann.

Es ist bevorzugt, wenn die Länge der Madenschraube so gewählt ist, dass sie bei Fehlen des abnehmbaren Konusaufsatzes am Prothesenteil mit konusförmigem Vorsprung aus der Durchgangsöffnung zur Durchführung des Sicherungselementes ins Innere des Prothesenteils mit konusförmiger Bohrung fallen kann. Die Madenschraube kann dabei in die konusförmige Bohrung des entsprechenden Prothesenteils oder in einen anderen Hohlraum fallen.

Sind die zwei Prothesenteile einer Prothese zusammengesetzt, d. h. die Konusverbindung zwischen den beiden Prothesenteilen ist hergestellt, ist nicht mehr erkennbar, ob sich ein Konusaufsatz an dem Prothesenteil mit dem konusförmigen Vorsprung befindet. Eine visuelle Überprüfung ist dann nicht mehr möglich, ohne die Konusverbindung wieder zu lösen. Indem die Länge der Madenschraube wie beschrieben gewählt wird, kann sichergestellt werden, dass im Falle des Fehlens des Konusaufsatzes dem Chirurg ein deutlicher Hinweis diesbezüglich gegeben wird. In diesem Falle wird sich das Sicherungselement bzw. die Madenschraube nämlich nicht festziehen lassen, sondern vielmehr in die konusförmige Bohrung des weiteren Prothesenteils oder eines vergleichbaren Hohlraums fallen. Dem Chirurg wird dadurch angezeigt, dass der Konusaufsatz fehlt und die Konusverbindung zwischen den beiden Prothesenteilen nicht gesichert ist.

Bei der vorbeschriebenen Prothese kann die konusförmige Bohrung in dem einen Prothesenteil ohne Weiteres dünnwandig ausgeführt sein. Die Durchgangsöffnung zur Durchführung des Sicherungselementes muss nämlich nicht durch die konusförmige Bohrung umgebende Wand führen, sondern kann vielmehr in einem anderen Bereich des Prothesenteils angeordnet sein, in dem ein Ausreißen der Durchgangsöffnung nicht zu erwarten ist. Die beschriebenen Vorteile ergeben sich selbstverständlich aber auch bei einer Prothese mit einer dickwandig ausgeführten, konusförmige Bohrung in dem weiteren Prothesenteil.

Bei dem Prothesenteil mit dem konusförmigen Vorsprung kann es sich bevorzugt um einen Prothesenschaft handeln, der Prothesenteil mit der konusförmigen Bohrung ist bevorzugt ein prothetisches Gelenk oder ein Teil eines prothetischen Gelenks.

Die Madenschraube weist bevorzugt eine kegelförmige Spitze auf, die mit der Ausnehmung am Konusaufsatz derart in Eingriff bringbar ist, dass eine Vorspannung in der Konusverbindung erzeugt wird. Die kegelförmige Spitze kann nach dem Prinzip der schiefen Ebene bei einer Verschiebung längs der Achse der Madenschraube (in der Regel durch Verdrehen der Madenschraube) und Zusammenwirken mit der Ausnehmung im Konusaufsatz eine Vorspannung in der Konusverbindung erzeugen, die eine Relativbewegung der beiden Prothesenteile verhindert. Die kegelförmige Spitze kann dabei mit einer Kante der Ausnehmung im Konusaufsatz zusammenwirken. Es ist aber auch möglich, dass die Ausnehmung eine zur kegelförmigen Spitze der Madenschraube komplementäre Gleitfläche aufweist.

Die Erfindung betrifft weiterhin einen Satz umfassend ein erstes erfindungsgemäßes Prothesenteil mit konusförmigem Vorsprung, sowie ein zweites und ein drittes Prothesenteil mit jeweils einer komplementären konusförmigen Bohrung. Das erste Prothesenteil kann also sowohl mit dem zweiten als auch dem dritten Teil eine Konusverbindung eingehen.

Der zweite Prothesenteil weist eine Durchgangsöffnung zur Durchführung eines Sicherungselementes auf, sodass das Sicherungselement bei dem eingesetzten ersten Prothesenteil in die seitliche Ausnehmung des Konusaufsatzes eingreifen kann. Die konusförmige Bohrung am zweiten Prothesenteil kann dünnwandig oder dickwandig ausgeführt sein.

Der dritte Prothesenteil weist eine Durchgangsöffnung zur Durchführung eines Sicherungselementes auf, sodass das Sicherungselement bei dem eingesetzten ersten Prothesenteil in die seitliche Ausnehmung des konusförmigen Vorsprungs eingreifen kann. Im dritten Prothesenteil ist ein Hohlraum zur Aufnahme des Konusaufsatzes am ersten Prothesenteil vorgesehen. Es ist aber auch möglich, dass ein entsprechender Hohlraum im dritten Prothesenteil nicht vorhanden ist. In diesem Fall muss dann der Konusaufsatz vom ersten Prothesenteil abgenommen werden, damit der erste und dritte Prothesenteil verbunden werden können.

Unabhängig davon, wie der dritte Prothesenteil ausgebildet ist, lässt sich der erste Prothesenteil wahlweise mit dem zweiten oder dritten Prothesenteil verbinden und durch ein Sicherungselement sichern. Der erste Prothesenteil ist also äußerst flexibel in seiner Verwendung, da er mit verschiedenen Ausgestaltungen von Prothesenteilen mit konusförmiger Bohrung verbunden werden kann.

Der Satz kann weiterhin noch wenigstens ein Sicherungselement aufweisen, wobei es sich bei dem Sicherungselement bevorzugt um eine Madenschraube handelt. Es ist weiter bevorzugt, dass die Durchgangsöffnungen im zweiten und dritten Prothesenteil wenigstens teilweise ein Innengewinde mit gleichem Durchmesser aufweisen, sodass ein Sicherungselement wahlweise mit dem zweiten oder dritten Prothesenteil verwendet werden kann.

Die Madenschraube weist bevorzugt eine kegelförmige Spitze auf, die mit der Ausnehmung am Konusaufsatz oder am konusförmigen Vorsprung derart in Eingriff bringbar ist, dass eine Vorspannung in der Konusverbindung erzeugt wird. Die kegelförmige Spitze kann nach dem Prinzip der schiefen Ebene bei einer Verschiebung längs der Achse der Madenschraube (in der Regel durch Verdrehen der Madenschraube) und Zusammenwirken mit der Ausnehmung im Konusaufsatz oder am konusförmigen Vorsprung eine Vorspannung in der Konusverbindung erzeugen, die eine Relativbewegung der beiden Prothesenteile verhindert. Die kegelförmige Spitze kann dabei mit einer Kante der Ausnehmung im Konusaufsatz oder am konusförmigen Vorsprung zusammenwirken. Es ist aber auch möglich, dass die jeweilige Ausnehmung eine zur kegelförmigen Spitze komplementäre Gleitfläche aufweist.

Für den Fachmann ist selbstverständlich, dass die einzelnen Aspekte der Erfindung, die in Zusammenhang mit dem Prothesenteil, der Prothese oder dem Satz von Prothesenteilen erläutert wurden, auch auf die jeweils anderen Gegenstände der Erfindung angewendet werden können.

Die Erfindung wird nachfolgend unter Bezugnahme auf die beigefügten Zeichnungen beispielhaft beschrieben. Es zeigen:
- Figur 1a-c:: verschiedene Ansichten eines Ausführungsbeispiels eines erfindungsgemäßen Prothesenteils;
- Figur 2a-c:: drei Ausführungsbeispiele weiterer Prothesenteile, mit denen sich das Prothesenteil aus Figur 1 verbinden lässt;
- Figur 3a,b:: ein Ausführungsbeispiel einer erfindungsgemäßen Prothese; und
- Figur 4:: ein weiteres Ausführungsbeispiel einer erfindungsgemäßen Prothese; und
- Figur 5:: ein Ausführungsbeispiel eines erfindungsgemäßen Satzes von Prothesenteilen.

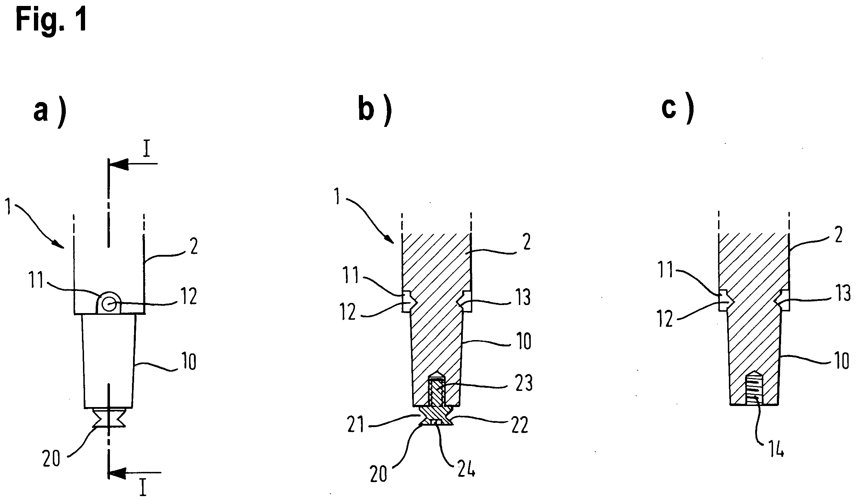

In Figur 1a-c ist ein erfindungsgemäßer Prothesenteil 1 dargestellt. Figur 1a zeigt dabei eine Seitenansicht, während Figuren 1b,c Schnittdarstellungen entlang der Schnittlinie I-I in Figur 1a zeigen.

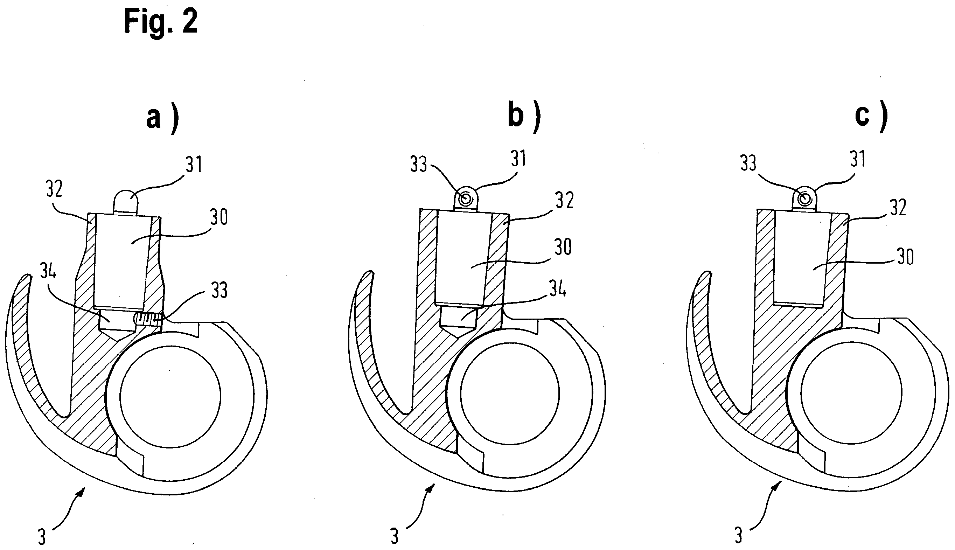

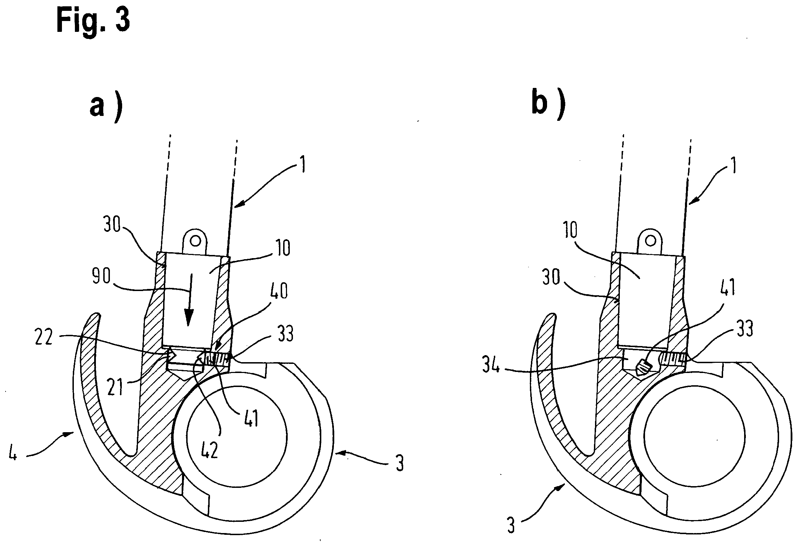

Der erfindungsgemäße Prothesenteil 1, bei dem es sich im dargestellten Ausführungsbeispiel um einen Prothesenschaft 2 handelt, der nur teilweise dargestellt ist, weist einen konusförmigen Vorsprung 10 auf. Mit diesem konusförmigen Vorsprung 10 ist es möglich, den Prothesenteil 1 mit einem weiteren Prothesenteil 3 (siehe Figur 2) zu verbinden. Eine entsprechende Konusverbindung ist bspw. in Figur 3 dargestellt und wird in diesem Zusammenhang noch erläutert werden.

Der Prothesenteil 1 weist weiterhin zwei Vertiefungen 11 auf, in die bspw. Positionierungszungen 31 eines weiteren Prothesenteils 3 (siehe Figur 2) eingreifen können, um so eine Verdrehsicherheit der Konusverbindung zu erreichen.

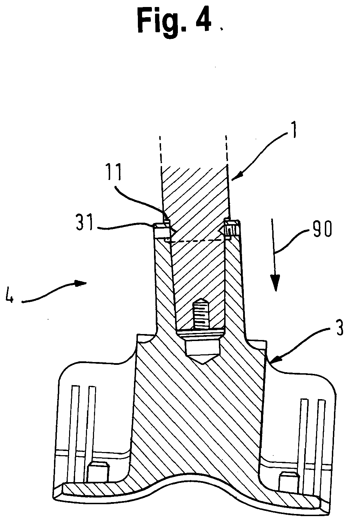

Auf dem Grund dieser Vertiefungen ist jeweils eine Ausnehmung 12 vorgesehen, in die ein Sicherungselement 40 (siehe Figur 4) eingreifen kann. Die Ausnehmungen 12 sind dabei als Sackloch mit spitz zulaufendem Grund 13 ausgebildet. Es ist aber selbstverständlich auch möglich, dass der Grund 13 eben und/oder geneigt ist.

Die Ausnehmungen 12 im dargestellten Ausführungsbeispiel werden eindeutig dem konusförmigen Vorsprung 10 zugeordnet, auch wenn sie nicht auf der konusförmig ausgestalteten Fläche des Vorsprungs 10 angeordnet sind. Zu dem konusförmigen Vorsprung gemäß der Erfindung gehören nämlich nicht nur die Bereiche, die tatsächlich konusförmig ausgestaltet sind, sondern auch solche Elemente und Merkmale, die elementar zur Bildung und Sicherung einer Konusverbindung sind.

An dem konusförmigen Vorsprung 10 ist weiterhin ein Konusaufsatz 20 vorgesehen. Dieser Konusaufsatz 20 ist abnehmbar ausgestaltet und rotationssymmetrisch geformt. Ist der Konusaufsatz 20 am konusförmigen Vorsprung 10 befestigt, so fällt seine Symmetrieachse mit der Achse des konusförmigen Vorsprungs 10 zusammen (vgl. Figuren 1a,b).

Der Konusaufsatz 20 weist eine umlaufende Nut 21 auf, deren Grund 22 spitz zulaufend ausgebildet ist. Es ist aber auch möglich, dass der Nutgrund eben und/oder geneigt ausgebildet ist. Außerdem weist der Konusaufsatz 20 noch einen schraubenförmigen Fortsatz 23 auf, mit dem er durch eine Gewindebohrung 14 am konusförmigen Vorsprung 10 befestigbar ist. Um das ggf. erforderliche Abnehmen des Konusaufsatzes 20 zu erleichtern, ist weiterhin eine Formvertiefung 24 für den Eingriff eines Innensechskantschlüssels vorgesehen. Durch die Verwendung eines entsprechenden Werkzeuges lässt sich der Konusaufsatz 20 einfach von dem Prothesenteil 1 bzw. dem konusförmigen Vorsprung 10 abnehmen. In Figur 1c ist das Prothesenteil 1 mit abgenommenem Konusaufsatz 20 dargestellt.

In Figur 2a-c sind beispielhaft drei weitere Prothesenteile 3 dargestellt, mit denen sich der Prothesenteil 1 aus Figur 1a-c verbinden lässt. Bei allen dargestellten weiteren Prothesenteilen 3 handelt es sich um eine femurale Komponente einer Kniegelenksprothese.

Alle Prothesenteile 3 aus Figur 2a-c weisen eine konusförmige Bohrung 30 auf, die komplementär zum konusförmigen Vorsprung 10 des Prothesenteils 1 aus Figur 1a-c ausgestaltet sind. Außerdem weisen sie Zungen 31 auf, die mit den Vertiefungen 11 am konusförmigen Vorsprung 10 des Prothesenteils 1 zusammenwirken können, um im zusammengesetzten Zustand der Prothesenteile 1 und 3 eine Verdrehsicherung zu erreichen. Aus darstellungstechnischen Gründen ist in Figur 2a-c lediglich eine Zunge 31 dargestellt.

Bei dem Prothesenteil 3 gemäß Figur 2a ist die konusförmige Bohrung 30 dünnwandig ausgestaltet, d. h. die die konusförmige Bohrung 30 umgebende Wand 32 weist eine zu geringe Wandstärke auf, als dass eine Durchgangsöffnung 33 zur Durchführung eines Sicherungselementes (nicht dargestellt) ausreichend sicher gegen ein Ausreißen wäre. Eine entsprechende Durchgangsöffnung 33 ist vielmehr in einem Bereich abseits der konusförmigen Bohrung 30 angeordnet, wo eine ausreichende Wandstärke vorliegt.

Die Durchgangsöffnung 33, die ein Innengewinde aufweist, endet in einem Hohlraum 34 am Boden der konusförmigen Bohrung 30. Der Hohlraum 34 ist dazu ausgebildet, den Konusaufsatz 20 am konusförmigen Vorsprung 10 des Prothesenteils 1 aus Figur 1a,b aufzunehmen. Ein durch die Durchführungsöffnung 33 hindurch geführtes Sicherungselement kann mit dem Konusaufsatz 20 zur Sicherung der Konusverbindung zwischen dem Prothesenteil 3 aus Figur 2a und einem Prothesenteil gemäß Figur 1a,b zusammenwirken. Dies wird später noch näher erläutert. Der Hohlraum 34 kann, wie dargestellt, besonders geformt sein. Es ist aber auch möglich, dass der Hohlraum 34 als Fortführung der konusförmigen Bohrung 30 ausgestaltet ist.

In Figur 2b ist ein weiterer Prothesenteil 3 mit konusförmiger Bohrung 30 dargestellt. Im Gegensatz zum Prothesenteil 3 aus Figur 2a ist hier die konusförmige Bohrung 30 jedoch dickwandig ausgeführt. Die konusförmige Bohrung 30 umgebene Wand 32 ist also ausreichend dick, um eine Durchführungsöffnung 33 zur Durchführung eines Sicherungselementes (nicht dargestellt) vorsehen zu können, ohne dass die Gefahr eines Ausreißens dieser Durchführungsöffnung 33 bestehen würde.

Bei dem Prothesenteil 3 gemäß Figur 2b sind zwei Durchgangsöffnungen 33 vorgesehen, die jeweils im Bereich einer Zunge 31 vorgesehen sind und ein dem Innengewinde der Durchgangsöffnung 33 des Prothesenteils 3 aus Figur 2a entsprechendes Gewinde aufweisen. Aus darstellungstechnischen Gründen ist in Figur 2b lediglich eine Zunge 31 bzw. eine Durchgangsöffnung 33 dargestellt.

Die Durchgangsöffnungen 33 sind demnach so angeordnet, dass ein durch eine der Durchgangsöffnungen 33 geführtes Sicherungselement (nicht dargestellt) in eine der Ausnehmungen 12 am konusförmigen Vorsprung 10 des Prothesenteils 1 aus Figur 1a-c eingreifen kann, um eine entsprechende Konusverbindung zu sichern.

Das Prothesenteil 3 gemäß Figur 2b weist einen Hohlraum 34 auf, der für die evtl. Aufnahme des Konusaufsatzes 20 des Prothesenteils 1 aus Figuren 1a,b ausgebildet ist. Auch wenn der Konusaufsatz 20 bei einem Prothesenteil 3 gemäß Figur 2b nicht zur Sicherung der Konusverbindung zwischen Prothesenteilen gemäß Figuren 1a,b und 2b benötigt wird, muss der Konusaufsatz 20 nicht zwingend vom konusförmigen Vorsprung 10 des Prothesenteils 1 gemäß Figur 1a,b abgenommen werden.

Das Prothesenteil 3 gemäß Figur 2c entspricht in weiten Teilen demjenigen aus Figur 2b, weshalb auf die diesbezüglichen Ausführungen verwiesen wird. Allerdings ist bei dem Prothesenteil 3 gemäß Figur 3c kein Hohlraum 34 (vgl. Figur 2b) für die Aufnahme des Konusaufsatzes 20 vorgesehen. Bevor das Prothesenteil 1 gemäß Figur 1a-c mit dem Prothesenteil 3 aus Figur 2c verbunden werden kann, muss zuerst der Konusaufsatz 20 vom Prothesenteil 1 abgenommen werden (vgl. Figur 1c).

In Figur 3a ist dargestellt, wie sich die Prothesenteile 1 und 3 aus Figuren 1a,b und 2a zu einer erfindungsgemäßen Prothese 4 zusammensetzen lassen. Der konusförmige Vorsprung 20 des einen Prothesenteils 1 ist dabei in die konusförmige Bohrung 30 des weiteren Prothesenteils 3 eingeführt. Es handelt sich also um eine klassische Konusverbindung zwischen den beiden Prothesenteilen 1, 3. Die Zungen 31 am weiteren Prothesenteil 3 greifen dabei in die Vertiefungen 11 des Prothesenteils 1 ein, sodass eine Verdrehsicherung gegeben ist.

Durch die Durchführungsöffnung 33 ist ein Sicherungselement 40 geführt. Bei dem Sicherungselement 40 handelt es sich um eine Madenschraube 41 mit einer kegelförmigen Spitze 42, die in das Gewinde der Durchführungsöffnung 33 eingreift. Auf der gegenüberliegenden Seite der Madenschraube 41 ist eine Formvertiefung vorgesehen (nicht dargestellt), in die ein Innensechskant eingreifen kann. Die Formvertiefung an der Madenschraube 41 kann bevorzugt die gleiche Nenngröße haben, wie die Formvertiefung 24 am Konusaufsatz 20.

Das Sicherungselement 40 ist derart weit durch die Durchführungsöffnung 33 hindurch geführt, dass es mit dem Konusaufsatz 20, der sich im Hohlraum 34 befindet, in Kontakt kommt. Dabei wirkt die kegelförmige Spitze 42 der Madenschraube 41 so mit dem Grund 22 der Ausnehmung 21 zusammen, dass eine Vorspannkraft in Richtung des Pfeils 90 in die Konusverbindung induziert wird.

Die Länge der Madenschraube 41 ist bei dem Ausführungsbeispiel in Figur 3 derart gewählt, dass die Madenschraube 41 bei Fehlen des Konusaufsatzes 20 aus der Durchgangsöffnung 33 ins Innere des Prothesenteils mit konusförmiger Bohrung, also in den Hohlraum 34 bzw. die konusförmige Bohrung 30 fällt. Entsprechendes ist in Figur 2b dargestellt. Indem die Madenschraube 41 aus der Durchgangsöffnung 33 ins Innere des Prothesenteils 3 fällt, wird dem operierenden Chirurgen angezeigt, dass die Konusverbindung zwischen den beiden Prothesenteilen 1, 3 nicht gesichert ist.

In Figur 4 ist dargestellt, wie sich das Prothesenteil 1 aus Figur 1c mit einem weiteren Prothesenteil 3 gemäß Figur 2b zu einer erfindungsgemäßen Prothese 4 verbinden lässt.

Der konusförmige Vorsprung 10 des einen Prothesenteils 1 ist dabei zur Bildung einer Konusverbindung in die konusförmige Bohrung 30 des anderen Prothesenteils 3 eingeführt, wobei die Zungen 31 in die Vertiefung 11 als Verdrehsicherung eingreifen.

Durch eine der beiden Durchgangsöffnungen 33 ist eine Madenschraube 41 als Sicherungselement 40 geführt. Die Madenschraube 41 weist dabei eine kegelförmige Spitze 42 auf, die mit dem Grund 13 der Ausnehmung 12 an dem konusförmigen Vorsprung 10 des Prothesenteils 1 so zusammenwirkt, dass eine Vorspannkraft in Richtung des Pfeils 90 erzeugt wird.

Im dargestellten Ausführungsbeispiel sind die Prothesenteile 1, 3 gemäß Figuren 1c und 2b miteinander verbunden. Es ist aber ebenso möglich, die Prothesenteile 1, 3 gemäß Figuren 1c und 2c oder Figuren 1a,b und 2b entsprechend miteinander zu verbinden.

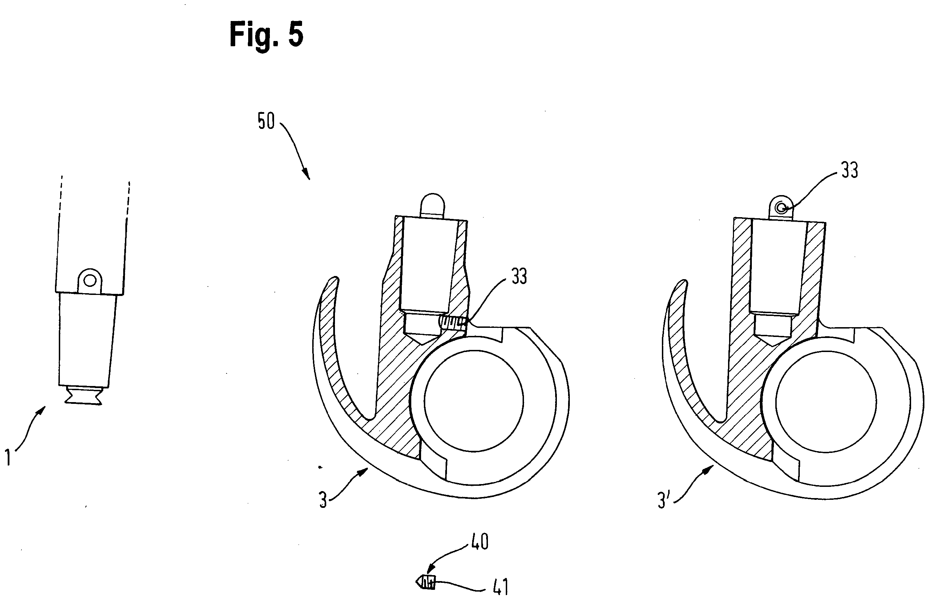

In Figur 5 ist ein erfindungsgemäßer Satz 50 von Prothesenteilen 1, 3, 3' dargestellt. Der erste Prothesenteil entspricht dabei demjenigen gemäß Figur 1, während der zweite und dritte Prothesenteil 3, 3' denjenigen aus Figuren 2a und 2b entsprechen. Der Satz 50 von Prothesenteilen 1, 3, 3' umfasst weiterhin eine Madenschraube 41 als Sicherungselement 40. Es ist vorgesehen, das die Durchgangsöffnungen 33 der Prothesenteile 3, 3' identische Innengewinde aufweisen, sodass die Madenschraube 41 sowohl mit dem einen Prothesenteil 3 als auch dem anderen Prothesenteil 3' verwendet werden kann. Für die Möglichkeiten der Verbindung der einzelnen Prothesenteile 1, 3, 3' untereinander wird auf die vorstehenden Ausführungen verwiesen.

## Patentansprüche

1. Prothesenteil (1) mit einem konusförmigen Vorsprung (10) zur Verbindung mit einem weiteren Prothesenteil (3) mit einer komplementären konusförmigen Bohrung (30), wobei der konusförmige Vorsprung (10) eine seitliche Ausnehmung (12) für den Eingriff eines Sicherungselementes (40) aufweist,
**dadurch gekennzeichnet, dass** das Prothesenteil (1) mit dem konusförmigen Vorsprung (10) einen abnehmbaren Konusaufsatz (20) aufweist, der eine seitliche Ausnehmung (21) für den Eingriff eines Sicherungselementes (40) aufweist.

2. Prothesenteil nach Anspruch 1, **dadurch gekennzeichnet, dass** die seitliche Ausnehmung (12) für den Eingriff eines Sicherungselementes (40) am konusförmigen Vorsprung (10) des Prothesenteils (1) als umlaufende Vertiefung oder Sackloch ausgebildet ist.

3. Prothesenteil nach Anspruch 1 oder 2, **dadurch gekennzeichnet, dass** der Konusaufsatz (20) rotationssymmetrisch, vorzugsweise zylindrisch, ausgebildet ist.

4. Prothesenteil nach Anspruch 3, **dadurch gekennzeichnet, dass** die Symmetrieachse des Konusaufsatzes (20) im zusammengesetzten Zustand des Prothesenteils (1) mit der Achse des konusförmigen Vorsprungs (10) zusammenfällt.

5. Prothesenteil nach einem der vorhergehenden Ansprüche, **dadurch gekennzeichnet, dass** die Ausnehmung (21) am Konusaufsatz (20) als umlaufende Vertiefung oder als Sackloch ausgebildet ist.

6. Prothesenteil nach einem der vorhergehenden Ansprüche, **dadurch gekennzeichnet, dass** der Konusaufsatz (20) über eine Schraubenverbindung abnehmbar mit dem konusförmigen Vorsprung (10) verbunden ist, wobei der Konusaufsatz (20) vorzugsweise einen schraubenförmigen Fortsatz (23), der konusförmige Vorsprung (10) vorzugsweise ein Sackloch (14) mit Innengewinde zur Bildung der Schraubenverbindung aufweisen.

7. Satz (50) umfassend ein erstes Prothesenteil (1) gemäß Anspruch 1 mit konusförmigen Vorsprung (10), ein zweites und ein drittes Prothesenteil (3, 3') mit jeweils einer komplementären konusförmigen Bohrung (30), wobei das zweite Prothesenteil (3) eine Durchgangsöffnung (33) zur Durchführung eines Sicherungselementes (40) aufweist, sodass das Sicherungselement (40) mit der seitlichen Ausnehmung (21) des abnehmbaren Konusaufsatzes (20) des in das zweite Prothesenteil (3) eingesetzten ersten Prothesenteils (1) in Eingriff bringbar ist, und wobei das dritte Prothesenteil (3') eine Durchgangsöffnung (33) zur Durchführung eines Sicherungselementes (40) aufweist, sodass das Sicherungselement (40) mit der seitlichen Ausnehmung (12) des konusförmigen Vorsprungs (10) des in das dritte Prothesenteil (3') eingesetzten ersten Prothesenteils (1) in Eingriff bringbar ist.

8. Satz nach Anspruch 7, **dadurch gekennzeichnet, dass** das Sicherungselement eine Madenschraube (41) ist und eine kegelförmige Spitze (42) aufweist, die wahlweise mit der Ausnehmung (21) am Konusaufsatz (20) oder der Ausnehmung (12) am konusförmigen Vorsprung (10) derart in Eingriff bringbar ist, dass eine Vorspannung in der Konusverbindung erzeugt wird.

## Claims

1. Prosthesis part (1) with a conical protrusion (10) for connection to a further prosthesis part (3) having a complementary conical bore (30), the conical protrusion (10) having a lateral recess (12) for the engagement of a securing element (40), **characterized in that** the prosthesis part (1) with the conical protrusion (10) has a removable cone attachment (20), which has a lateral recess (21) for the engagement of a securing element (40).

2. Prosthesis part according to Claim 1, **characterized in that** the lateral recess (12) for the engagement of a securing element (40) on the conical protrusion (10) of the prosthesis part (1) is designed as a circumferential depression or blind hole.

3. Prosthesis part according to Claim 1 or 2, **characterized in that** the cone attachment (20) is rotationally symmetrical, preferably cylindrical.

4. Prosthesis part according to Claim 3, **characterized in that** the axis of symmetry of the cone attachment (20) in the assembled state of the prosthesis part (1) coincides with the axis of the conical protrusion (10).

5. Prosthesis part according to one of the preceding claims, **characterized in that** the recess (21) on the cone attachment (20) is designed as a circumferential depression or as a blind hole.

6. Prosthesis part according to one of the preceding claims, **characterized in that** the cone attachment (20) is connected removably to the conical protrusion (10) by a screw connection, the cone attachment (20) preferably having a screw-shaped extension (23), and the conical protrusion (10) preferably having a blind hole (14) with an internal thread for forming the screw connection.

7. Kit (50) comprising a first prosthesis part (1) according to Claim 1 with a conical protrusion (10), a second prosthesis part (3) and a third prosthesis part (3'), each having a complementary conical bore (30), wherein the second prosthesis part (3) has a through-opening (33) allowing the passage of a securing element (40), such that the securing element (40) can be brought into engagement with the lateral recess (21) of the removable cone attachment (20) of the first prosthesis part (1) inserted into the second prosthesis part (3), and wherein the third prosthesis part (3') has a through-opening (33) allowing the passage of a securing element (40), such that the securing element (40) can be brought into engagement with the lateral recess (12) of the conical protrusion (10) of the first prosthesis part (1) inserted into the third prosthesis part (3').

8. Kit according to Claim 7, **characterized in that** the securing element is a headless screw (41) and has a tapered tip (42) which can optionally be brought into engagement with the recess (21) on the cone attachment (20) or with the recess (12) on the conical protrusion (10) in such a way that pre-tensioning is generated in the cone connection.

## Revendications

1. Partie de prothèse (1) comprenant une saillie de forme conique (10) pour la connexion à une autre partie de prothèse (3) avec un alésage complémentaire de forme conique (30), la saillie de forme conique (10) présentant un évidement latéral (12) pour l'engagement d'un élément de fixation (40),
**caractérisée en ce que** la partie de prothèse (1) présente avec la saillie de forme conique (10) un chapeau conique amovible (20) qui présente un évidement latéral (21) pour l'engagement d'un élément de fixation (40).

2. Partie de prothèse selon la revendication 1, **caractérisée en ce que** l'évidement latéral (12) est réalisé sous forme de renfoncement périphérique ou de trou borgne pour l'engagement d'un élément de fixation (40) au niveau de la saillie de forme conique (10) de la partie de prothèse (1).

3. Partie de prothèse selon la revendication 1 ou 2, **caractérisée en ce que** le chapeau conique (20) est réalisé avec une symétrie de révolution, de préférence sous forme cylindrique.

4. Partie de prothèse selon la revendication 3, **caractérisée en ce que** l'axe de symétrie du chapeau conique (20), dans l'état assemblé de la partie de prothèse (1), coïncide avec l'axe de la saillie de forme conique (10).

5. Partie de prothèse selon l'une quelconque des revendications précédentes, **caractérisée en ce que** l'évidement (21) au niveau du chapeau conique (20) est réalisé sous forme de renfoncement périphérique ou sous forme de trou borgne.

6. Partie de prothèse selon l'une quelconque des revendications précédentes, **caractérisée en ce que** le chapeau conique (20) est connecté par le biais d'une connexion par vissage de manière amovible à la saillie de forme conique (10), le chapeau conique (20) présentant de préférence un prolongement de forme hélicoïdale (23), la saillie de forme conique (10) présentant de préférence un trou borgne (14) avec un filetage interne pour former la connexion par vissage.

7. Ensemble (50) comprenant une première partie de prothèse (1) selon la revendication 1 avec une saillie de forme conique (10), une deuxième et une troisième partie de prothèse (3, 3') avec à chaque fois un alésage complémentaire de forme conique (30), la deuxième partie de prothèse (3) présentant une ouverture de passage (33) pour le passage d'un élément de fixation (40) de telle sorte que l'élément de fixation (40) puisse être amené en prise avec l'évidement latéral (21) du chapeau conique amovible (20) de la première partie de prothèse (1) insérée dans la deuxième partie de prothèse (3), et la troisième partie de prothèse (3') présentant une ouverture de passage (33) pour le passage d'un élément de fixation (40) de telle sorte que l'élément de fixation (40) puisse être amené en prise avec l'évidement latéral (12) de la saillie de forme conique (10) de la première partie de prothèse (1) insérée dans la troisième partie de prothèse (3').

8. Ensemble selon la revendication 7, **caractérisé en ce que** l'élément de fixation est une vis sans tête (41) et présente une pointe de forme conique (42) qui peut être amenée en prise de manière sélective avec l'évidement (21) au niveau du chapeau conique (20) ou avec l'évidement (12) au niveau de la saillie de forme conique (10) de telle sorte que l'on obtienne une précontrainte dans la connexion conique.
